# EUROPÄISCHE PATENTANMELDUNG

(11) **EP 4 473 842 A1**
(43) Veröffentlichungstag der Anmeldung: **11.12.2024**
(21) Anmeldenummer: 24156186.9
(22) Anmeldetag: 07.02.2024
(51) Int. Cl.: A23L 3/00, A23L 3/04

(54) **VERFAHREN ZUR REGELUNG EINER TRANSPORTGESCHWINDIGKEIT IN EINEM PASTEUR UND VORRICHTUNG ZUM AUSFÜHREN DES VERFAHRENS**

(30) Priorität: 06.06.2023 DE 102023114812
(71) Anmelder: Krones AG, 93073 Neutraubling (DE)
(72) Erfinder: Muenzer, Jan, 93073 Neutraubling (DE); Kaatz, Stefan, 93073 Neutraubling (DE); Schmuck, Jesse, 93073 Neutraubling (DE)
(74) Vertreter: Grünecker Patent- und Rechtsanwälte PartG mbB

(57) **Zusammenfassung**

Die Erfindung betrifft ein Verfahren zur Regelung einer Transportgeschwindigkeit in einem Pasteur, wobei das Verfahren eine Regelung der Transportgeschwindigkeit durch ein Verändern, wie Reduzieren oder Erhöhen, der Transportgeschwindigkeit, ein entsprechendes erstes Verändern, wie Reduzieren oder Erhöhen, einer ersten Anzahl von Pasteurisierungszonen (22, 23, 24, 39, 40, 65, 76, 77, 108, 109) um eine zweite Anzahl und ein entsprechendes zweites Verändern, wie Erhöhen oder Reduzieren, einer dritten Anzahl von Kühlzonen (25, 26, 27, 28, 29, 41, 42, 43, 44, 45, 46, 66, 67, 68, 69, 70, 71, 72, 78, 79, 110, 111) um die zweite Anzahl umfasst. Weiter betrifft die Erfindung eine Vorrichtung zum Ausführen des Verfahrens, wobei die Vorrichtung umfasst: einen Pasteur mit einer ersten Anzahl von Pasteurisierungszonen und einer dritten Anzahl von Kühlzonen, eine Transportvorrichtung, die ausgebildet ist, Behälter mit einer Transportgeschwindigkeit in eine Transportrichtung durch den Pasteur zu transportieren, und eine Steuerungsvorrichtung eingerichtet zum Verändern, wie Reduzieren oder Erhöhen, der Transportgeschwindigkeit, entsprechendes erstes Verändern, wie Reduzieren oder Erhöhen, der ersten Anzahl von Pasteurisierungszonen um eine zweite Anzahl und entsprechendes zweites Verändern, wie Erhöhen oder Reduzieren, der dritten Anzahl von Kühlzonen um die zweite Anzahl.

## Beschreibung

Die Erfindung betrifft ein Verfahren zur Regelung einer Transportgeschwindigkeit in einem Pasteur und eine Vorrichtung zum Ausführen des Verfahrens gemäß den unabhängigen Ansprüchen.

### Stand der Technik

EP 2 833 742 B1 offenbart einen Pasteur mit einem pasteurinternen Transportelement zum Bewegen von mit Füllgut gefüllten Behältern durch mehrere in Transportrichtung aufeinander folgende Zonen, in denen die Behälter mit einem flüssigen Behandlungsmedium mit unterschiedlichen Behandlungstemperaturen beaufschlagt werden. Die Behandlungstemperaturen steigen in einer Vorwärmzonen bildenden Gruppe von Zonen in der Transportrichtung von Zone zu Zone stufenförmig an. In wenigstens einer folgenden Pasteurisierzone entspricht die Behandlungstemperatur der Pasteurisiertemperatur und in nachfolgenden Kühlzonen nimmt in Transportrichtung von Zone zu Zone die Behandlungstemperatur stufenförmig ab. Die Transportgeschwindigkeit des Transportelements ist kontinuierlich steuer- oder regelbar. Bei Erhöhung der Transportgeschwindigkeit wird die Anzahl der als Vorwärmzonen dienenden Zonen bei gleichzeitiger Erhöhung der Behandlungstemperatur in diesen Zonen verringert und die Anzahl der als Pasteurisierzonen dienenden Zonen wird erhöht. Bei einer Reduzierung der Transportgeschwindigkeit wird die Anzahl der als Vorwärmzonen dienenden Zonen bei teilweiser Reduzierung der Behandlungstemperaturen erhöht und die Anzahl der als Pasteurisierzonen wirkenden Zonen reduziert wird.

### Aufgabe

Die Aufgabe der Erfindung ist es, ein Verfahren zur Regelung einer Transportgeschwindigkeit in einem Pasteur und eine Vorrichtung zum Ausführen des Verfahrens zur Verfügung zu stellen, die das Erreichen einer gewünschten Pasteurisierung ermöglichen können.

### Lösung

Die Aufgabe wird gelöst durch das Verfahren zur Regelung einer Transportgeschwindigkeit in einem Pasteur und die Vorrichtung zum Ausführen des Verfahrens gemäß den unabhängigen Ansprüchen. Weitere Ausführungsformen sind in den Unteransprüchen offenbart.

Das erfindungsgemäße Verfahren zur Regelung einer Transportgeschwindigkeit in einem Pasteur umfasst eine Regelung der Transportgeschwindigkeit durch ein Verändern, wie ein Reduzieren oder Erhöhen, der Transportgeschwindigkeit, entsprechendes erstes Verändern, wie Reduzieren oder Erhöhen, einer ersten Anzahl von Pasteurisierungszonen des Pasteurs um eine zweite Anzahl und entsprechendes zweites Verändern, wie Erhöhen oder Reduzieren, einer dritten Anzahl von Kühlzonen des Pasteurs um die zweite Anzahl.

Das Verändern der Transportgeschwindigkeit kann kontinuierlich erfolgen. Beispielsweise kann vorgesehen sein, dass kein stufenweises Verändern der Transportgeschwindigkeit erfolgt.

Das Verändern der Transportgeschwindigkeit kann ausgehend von jeder Transportgeschwindigkeit (wobei jede Transportgeschwindigkeit hier im möglichen Bereich des Pasteurs zu sehen sein kann) erfolgen, beispielsweise kontinuierlich. Beispielsweise kann vorgesehen sein, dass kein stufenweises Verändern der Transportgeschwindigkeit erfolgt.

Die Kühlzonen können in einer Transportrichtung von Behältern durch den Pasteur stromab der Pasteurisierungszonen angeordnet sein. Die Behälter können mittels einer Transportvorrichtung durch den Pasteur transportiert werden und können so die Pasteurisierungs- und Kühlzonen passieren. In den Pasteurisierungs- und Kühlzonen kann Behandlungsmedium mit unterschiedlichen Temperaturen auf die Behälter mittels jeweiliger Spritzvorrichtungen aufgespritzt werden.

Beispielsweise kann bei einem Reduzieren der Transportgeschwindigkeit die in der Transportrichtung letzte Pasteurisierungszone zu einer Kühlzone werden (beispielsweise durch eine entsprechende Anpassung der Temperatur des Behandlungsmediums). Die in der Transportrichtung stromauf der letzten Pasteurisierungszone, die zur Kühlzone werden kann, befindlichen Pasteurisierungszonen können weiter als Pasteurisierungszonen betrieben werden. Beispielsweise kann in ihnen die Pasteurisierungstemperatur erhöht werden.

Die Temperatur zumindest der letzten Pasteurisierungszone, die zur Kühlzone werden kann, kann beim Reduzieren der Transportgeschwindigkeit angepasst werden. So kann erreicht werden, dass in den Behältern vorhandenes Produkt trotz der reduzierten Transportgeschwindigkeit ausreichend pasteurisiert werden kann, also beispielsweise eine vorgegebene Anzahl von Pasteurisierungseinheiten aufnehmen kann. Eine Unter- oder Überpasteurisierung kann vermieden werden. Zudem kann durch eine Umwandlung der letzten Pasteurisierungszone in eine Kühlzone und Beibehaltung von in Transportrichtung stromauf befindlichen Pasteurisierungseinheiten eine Pasteurisierung des Produkts bei niedrigen Temperaturen vermieden werden.

Die Reduzierung der Transportgeschwindigkeit kann einen Stopp des Pasteurs ausschließen, also eine Reduzierung auf eine Geschwindigkeit Null. Die Reduzierung der Transportgeschwindigkeit kann eine Rückwärtsbewegung ausschließen, also einen Transport von Behältern entgegen der Transportrichtung.

Da die Regelung der Transportgeschwindigkeit durch eine Veränderung der Anzahl der Pasteurisierungszonen und der Kühlzonen während des Betriebs, beispielsweise kontinuierlich, erfolgen kann, sind beispielsweise keine Folgestopps erforderlich, um gegebenenfalls eine gewünschte Anzahl von Pasteurisierungseinheiten erreichen zu können.

Beispielsweise ist auch keine Vorhersage erforderlich, ob eventuell ein Reduzieren oder ein Erhöhen der Transportgeschwindigkeit erfolgen wird, um eine Regelung vornehmen zu können. Das Verfahren, wie hier, weiter oben oder weiter unten beschrieben, kann ohne eine beispielsweise zeitliche) Voraussage für eine Regelung oder ein Verändern der Transportgeschwindigkeit durchgeführt werden.

Das Verfahren kann weiter ein Beibehalten einer vierten Anzahl von Anwärmzonen des Pasteurs umfassen. Die Anwärmzonen können in einer Transportrichtung von Behältern durch den Pasteur stromauf der Pasteurisierungszonen angeordnet sein. Beispielsweise können die Anwärmzonen in Transportrichtung der Behälter eine stufenweise ansteigende Behandlungstemperatur eines Behandlungsmediums aufweisen. Das Behandlungsmedium kann Wasser mit der jeweiligen Behandlungstemperatur sein. Bei der Reduzierung der Transportgeschwindigkeit kann eine Erhöhung der Temperaturen des Behandlungsmediums in den einzelnen Anwärmzonen vorgesehen sein, wobei der stufenweise Temperaturanstieg von Anwärmzone zu Anwärmzone beibehalten werden kann. Bei der Erhöhung der Transportgeschwindigkeit kann eine Reduzierung der Temperaturen des Behandlungsmediums in den einzelnen Anwärmzonen vorgesehen sein, wobei der stufenweise Temperaturanstieg von Anwärmzone zu Anwärmzone beibehalten werden kann.

Das Reduzieren der ersten Anzahl von Pasteurisierungszonen des Pasteurs um die zweite Anzahl und das Erhöhen der dritten Anzahl von Kühlzonen des Pasteurs um die zweite Anzahl kann ein kontinuierliches Absenken einer ersten Temperatur in der letzten Pasteurisierungszone umfassen, wodurch die letzte Pasteurisierungszone zu einer ersten Kühlzone werden kann.

Das kontinuierliche Absenken einer ersten Temperatur in der letzten Pasteurisierungszone kann eine Umschaltung einer Versorgung einer Spritzungsvorrichtung in der letzten Pasteurisierungszone mit Behandlungsmedium aus der letzten Pasteurisierungszone in eine Versorgung der Spritzungsvorrichtung mit Behandlungsmedium aus der ersten Kühlzone und eine Umschaltung einer Versorgung einer Spritzungsvorrichtung in der letzten Aufwärmzone mit Behandlungsmedium aus der ersten Kühlzone in eine Versorgung der Spritzungsvorrichtung mit Behandlungsmedium aus der letzten Pasteurisierungszone umfassen.

Durch die Einbindung der letzten Pasteurisierungszone als Spritzkaskade mit der ersten Kühlzone (diejenige Kühlzone, die in Transportrichtung stromab der letzten Pasteurisierungszone angeordnet sein kann) in eine Rekuperation der Aufwärm- und Kühlzonen kann die gewünschte Temperaturabsenkung in der letzten Pasteurisierungszone erfolgen. Die so abgeführte Wärme aus der letzten Pasteurisierungszone kann der letzten Aufwärmzone zugeführt.

Je mehr die Transportgeschwindigkeit reduziert wird, umso mehr Pasteurisierungszonen können nach und nach in die Spritzkaskade eingebunden und zu Kühlzonen werden.

Das Verfahren kann weiter ein Unterbrechen einer separaten Zufuhr von Warmwasser in die letzte Pasteurisierungszone umfassen. Das warme Behandlungsmedium kann durch einen Wärmetauscher bereitgestellt werden.

Das Reduzieren der ersten Anzahl von Pasteurisierungszonen des Pasteurs um die zweite Anzahl und das Erhöhen der dritten Anzahl von Kühlzonen des Pasteurs um die zweite Anzahl kann mittels Regeln von mehreren regelbaren Ventilen erfolgen. Beispielsweise können die mehreren regelbaren Ventile in einer Druckverrohrung von Spritzvorrichtungen angeordnet sein. Beispielsweise können die mehreren regelbaren Ventile Klappenventile umfassen.

Durch das Vorsehen der Ventile in der Druckverrohrung der entsprechenden Spritzvorrichtungen kann ein ausreichender Druck des Behandlungsmediums und damit ein einheitliches Spritzbild der Spritzvorrichtungen gewährleistet werden.

Das Reduzieren der Transportgeschwindigkeit kann kontinuierlich, beispielsweise stufenlos, erfolgen oder das Reduzieren der Transportgeschwindigkeit kann in 0,5%-, 1%- oder 1,5%-Schritten erfolgen.

Das Verändern kann ein Erhöhen der Transportgeschwindigkeit, das entsprechende erste Verändern ein Erhöhen der ersten Anzahl von Pasteurisierungszonen des Pasteurs um die zweite Anzahl und das entsprechende zweite Verändern ein Reduzieren der dritten Anzahl von Kühlzonen des Pasteurs um die zweite Anzahl umfassen.

Das Erhöhen der ersten Anzahl von Pasteurisierungszonen des Pasteurs um die zweite Anzahl und das Erhöhen der dritten Anzahl von Kühlzonen des Pasteurs um die zweite Anzahl kann einkontinuierliches Erhöhen einer ersten Temperatur in der ersten Kühlzone umfassen, wodurch die erste Kühlzone zu einer letzten Pasteurisierungszone werden kann.

Das Verfahren kann ein Reduzieren der Transportgeschwindigkeit und dann ein Erhöhen der Transportgeschwindigkeit oder umgekehrt umfassen. Zwischen dem Reduzieren und Erhöhen bzw. Erhöhen und Reduzieren der Transportgeschwindigkeit kann die Transportgeschwindigkeit beispielsweise einen konstanten Wert für eine Zeitdauer aufweisen. Die Zeitdauer braucht nicht vorherbestimmt sein. Sie kann sich beispielsweise durch die Regelung ergeben.

Eine Vorrichtung zum Ausführen des Verfahrens, wie oben oder weiter unten beschrieben, umfasst einen Pasteur mit einer ersten Anzahl von Pasteurisierungszonen und einer dritten Anzahl von Kühlzonen, eine Transportvorrichtung, die ausgebildet ist, Behälter mit einer Transportgeschwindigkeit in eine Transportrichtung durch den Pasteur zu transportieren, und eine Steuerungsvorrichtung eingerichtet zum Verändern, wie Reduzieren oder Erhöhen, der Transportgeschwindigkeit, entsprechendes erstes Verändern, wie Reduzieren oder Erhöhen, der ersten Anzahl von Pasteurisierungszonen des Pasteurs um eine zweite Anzahl und entsprechendes zweites Verändern, wie Erhöhen oder Reduzieren, der dritten Anzahl von Kühlzonen des Pasteurs um die zweite Anzahl.

Die Kühlzonen können in einer Transportrichtung von Behältern durch den Pasteur stromab der Pasteurisierungszonen angeordnet sein. Die Behälter können mittels einer Transportvorrichtung durch den Pasteur transportiert werden und können so die Pasteurisierungs- und Kühlzonen passieren. In den Pasteurisierungs- und Kühlzonen kann Behandlungsmedium mit unterschiedlichen Temperaturen auf die Behälter mittels jeweiliger Spritzvorrichtungen aufgespritzt werden.

Der Pasteur kann eine vierte Anzahl von Anwärmzonen umfassen.

Die Anwärmzonen können in einer Transportrichtung von Behältern durch den Pasteur stromauf der Pasteurisierungszonen angeordnet sein. Beispielsweise können die Anwärmzonen in Transportrichtung der Behälter eine stufenweise ansteigende Behandlungstemperatur eines Behandlungsmediums aufweisen. Das Behandlungsmedium kann Wasser mit der jeweiligen Behandlungstemperatur sein. Bei der Reduzierung der Transportgeschwindigkeit kann eine Erhöhung der Temperaturen des Behandlungsmediums in den einzelnen Anwärmzonen vorgesehen sein, wobei der stufenweise Temperaturanstieg von Anwärmzone zu Anwärmzone beibehalten werden kann. Bei der Erhöhung der Transportgeschwindigkeit kann eine Reduzierung der Temperaturen des Behandlungsmediums in den einzelnen Anwärmzonen vorgesehen sein, wobei der stufenweise Temperaturanstieg von Anwärmzone zu Anwärmzone beibehalten werden kann.

Die Vorrichtung kann mehrere regelbare Ventile umfassen.

Das Reduzieren oder Erhöhen der ersten Anzahl von Pasteurisierungszonen des Pasteurs um die zweite Anzahl und das Erhöhen oder das Reduzieren der dritten Anzahl von Kühlzonen des Pasteurs um die zweite Anzahl kann mittels Regeln der mehreren regelbaren Ventile erfolgen.

Die mehreren regelbaren Ventile können in einer Druckverrohrung von Spritzvorrichtungen angeordnet sein. Beispielsweise können die mehreren regelbaren Ventile Klappenventile umfassen.

Die Vorrichtung kann weiter eine Umschaltvorrichtung zur Umschaltung einer Versorgung einer Spritzungsvorrichtung in der letzten Pasteurisierungszone mit Behandlungsmedium aus der letzten Pasteurisierungszone in eine Versorgung der Spritzungsvorrichtung mit Behandlungsmedium aus der ersten Kühlzone und weiter eine Umschaltvorrichtung zur Umschaltung einer Versorgung einer Spritzungsvorrichtung in der letzten Aufwärmzone mit Behandlungsmedium aus der ersten Kühlzone in eine Versorgung der Spritzungsvorrichtung mit Behandlungsmedium aus der letzten Pasteurisierungszone umfassen. Die Umschaltvorrichtungen können die mehreren regelbaren Ventile umfassen. Die Umschaltvorrichtung kann beispielsweise bei einer Reduzierung der Transportgeschwindigkeit zum Einsatz kommen, wenn die erste Anzahl von Pasteurisierungszonen des Pasteurs um die zweite Anzahl reduziert und die dritte Anzahl von Kühlzonen des Pasteurs um die zweite Anzahl erhöht werden soll.

Die Vorrichtung kann weiter einen Wärmetauscher zum Wärmen von Behandlungsmedium umfassen. Das erwärmte Behandlungsmedium kann der letzten Pasteurisierungszone zugeführt werden.

Die Vorrichtung kann weiter einen weiteren Wärmetauscher zum Kühlen von Behandlungsmedium umfassen.

### Kurze Figurenbeschreibung

Die beigefügten Figuren stellen beispielhaft zum besseren Verständnis und zur Veranschaulichung Aspekte und/oder Ausführungsbeispiele der Erfindung dar. Es zeigt:
Figur 1 ein Diagramm mit Temperaturkurven einer Pasteurisation und einer Kurve der aufgenommenen Pasteurisierungseinheiten bei 100% Transportgeschwindigkeit,
Figur 2 ein Diagramm mit Temperaturkurven einer Pasteurisation und einer Kurve der aufgenommenen Pasteurisierungseinheiten bei 80% Transportgeschwindigkeit,
Figur 3 ein Diagramm mit Temperaturkurven einer Pasteurisation und einer Kurve der aufgenommenen Pasteurisierungseinheiten bei 50% Transportgeschwindigkeit,
Figur 4 eine Schaltanordnung einer Spritzkaskade bei 100% Transportgeschwindigkeit,
Figur 5 eine Schaltanordnung der Spritzkaskade des in der Figur 4 beschriebenen Pasteurs, wobei die zweite Pasteurisierungszone gekühlt wird,
Figur 6 eine Schaltanordnung der Spritzkaskade des in der Figur 4 beschriebenen Pasteurs, wobei die zweite Pasteurisierungszone mittels der ersten Kühlzone gekühlt wird,
Figur 7 eine Schaltanordnung für eine Verschiebung einer Rekuperationszone bei 100% Transportgeschwindigkeit,
Figur 8 eine Schaltanordnung für eine Verschiebung der Rekuperationszone bei 80% Transportgeschwindigkeit und
Figur 9 eine Schaltanordnung für eine Verschiebung der Rekuperationszone bei 50% Transportgeschwindigkeit.

### Ausführliche Figurenbeschreibung

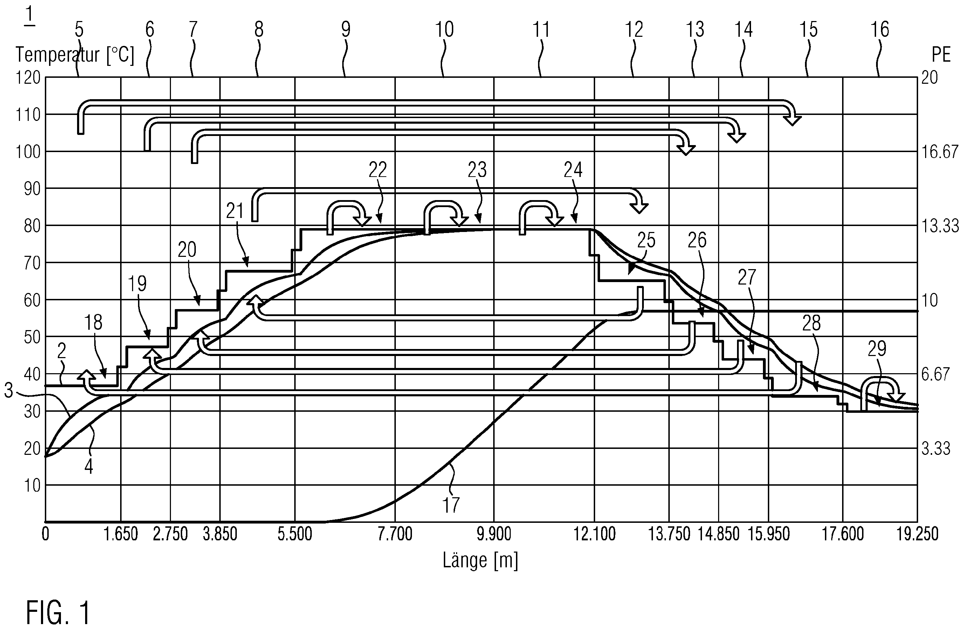

Die Figur 1 zeigt ein Diagramm 1 mit Temperaturkurven 2, 3, 4 einer Pasteurisation und einer Kurve 17 der aufgenommenen Pasteurisierungseinheiten PE bei 100% Transportgeschwindigkeit. 100% Transportgeschwindigkeit kann hierbei eine Nenntransportgeschwindigkeit sein, d.h. kann der Transportgeschwindigkeit während eines Normalbetriebs des Pasteurs entsprechen.

Die Pasteurisation kann in einem Pasteur durchgeführt werden, durch den mit Produkt befüllte Behälter mit der Transportgeschwindigkeit, beispielsweise angeordnet auf einer Transportvorrichtung, für einen Pasteurisationsprozess in eine Transportrichtung hindurchtransportiert werden können. Der beschriebene Pasteur umfasst insgesamt zwölf Zonen 5-16, die von den Behältern durchlaufen werden. Durch die jeweilige in einer Zone vorherrschende Temperatur des Behandlungsmediums, ergibt sich eine Einteilung in Aufwärmzone, Pasteurisierungszone oder Kühlzone.

Bei 100% Transportgeschwindigkeit dienen die ersten vier Zonen 5, 6, 7, 8 als Anwärmzonen 18, 19, 20, 21, die fünfte, sechste und siebte Zone 9, 10, 11 dienen jeweils als Pasteurisierungszone 22, 23, 24 und die achte bis zwölfte Zone 12, 13, 14, 15, 16 dienen jeweils als Kühlzone 25, 26, 27, 28, 29.

Im Pasteur werden zunächst nacheinander die vier Anwärmzonen 18-21 passiert, die von der ersten Zone 5 zur zweiten Zone 6 zur dritten Zone 7 und zur vierten Zone 8 stufenweise ansteigende Behandlungstemperaturen eines Behandlungsmediums aufweisen. Das Behandlungsmedium kann Wasser mit der jeweiligen Behandlungstemperatur sein, das mittels in den jeweiligen Zonen angeordneten Spritzvorrichtungen auf die Behälter aufgespritzt werden kann. In der ersten Zone 5 kann eine Temperatur des Behandlungsmediums, das auf die Behälter aufgespritzt werden kann, bei 36,6 °C liegen, in der zweiten Zone 6 bei 47, 2 °C, in der dritten Zone 7 bei 57, 2 °C und in der vierten Zone 8 bei 67,7 °C.

Nach den vier Anwärmzonen 18-21 können die Behälter die drei Pasteurisierungszonen 22-24 passieren, in denen die Behandlungstemperaturen des Behandlungsmediums in der fünften Zone 9, sechsten Zone 11 und siebten Zone 12 einer Pasteurisierungstemperatur entsprechen, die beispielsweise 78,8 °C betragen kann. An der Kurve 17 der aufgenommenen Pasteurisierungseinheiten PE ist die Zunahme der aufgenommenen Pasteurisierungseinheiten PE zu erkennen, die in der ersten Pasteurisierungszone 22 beginnt und sich in der zweiten Pasteurisierungszone 23 und der dritten Pasteurisierungszone 24 kontinuierlich fortsetzt. Die aufgenommenen Pasteurisierungseinheiten sind dabei in der Einheit PE über der im Pasteur zurückgelegten Länge in Meter angegeben.

Nach den Pasteurisierungszonen 22-24 können die Behälter die fünf Kühlzonen 25-29 passieren, wobei die Behandlungstemperaturen des Behandlungsmedium von der achten Zone 12 zur neunten Zone 13 zur zehnten Zone 14 zur elften Zone 15 und zur zwölften Zone 16 stufenförmig abnimmt. In der achten Zone 12 kann eine Temperatur des Behandlungsmediums, das auf die Behälter aufgespritzt werden kann, bei 65,0 °C liegen, in der neunten Zone 13 bei 53,7 °C, in der zehnten Zone 14 bei 43,8 °C, in der elften Zone 15 bei 33,9 °C und in der zwölften Zone 16 bei 29,9 °C. In der ersten Kühlzone 25 können durch die im Behälter vorherrschenden Temperaturen zu Beginn weiter Pasteurisierungseinheiten PE aufgenommen werden, erst gegen Ende der ersten Kühlzone 25 bleibt dann die Anzahl der aufgenommenen Pasteurisierungseinheiten konstant. Die nach der Pasteurisierung eines Behälters erreichten Pasteurisierungseinheiten PE können einen Wert von 9,5 haben.

In dem Diagramm ist der Kurvenverlauf der Behandlungstemperatur für die Spritzvorrichtungen mit dem Bezugszeichen 2 gekennzeichnet. Die Behandlungstemperatur ist dabei in Grad Celsius über der im Pasteur zurückgelegten Länge in Meter angegeben.

Zudem ist der Temperaturverlauf des Produkts im Innern der Behälter, an einem Punkt in einem Drittel der Höhe der Behälter von Boden aus gemessen, in der Kurve mit dem Bezugszeichen 4 angegeben. Die Produkttemperatur ist dabei in Grad Celsius über der im Pasteur zurückgelegten Länge in Meter angegeben

Weiter ist mit dem Bezugszeichen 3 der Kurvenverlauf der mittleren Temperatur dargestellt. Die mittlere Temperatur ist dabei in Grad Celsius über der im Pasteur zurückgelegten Länge in Meter angegeben.

Für die Verteilung des Behandlungsmediums zu den Spritzvorrichtungen der jeweiligen Zonen des Pasteurs ist vorgesehen, dass Behandlungsmedium aus der achten Zone 12 der Spritzvorrichtung der vierten Zone 8 zugeführt wird. Als Behandlungsmedium aus einer Zone ist solches Behandlungsmedium zu verstehen, das beispielsweise nach einem Aufspritzen auf Behälter unterhalb dieser Zone in einem Becken, oder dergleichen, gesammelt wird und von dort aus über ein entsprechendes Rohrleitungssystem einer Spritzvorrichtung zugeführt werden kann.

Behandlungsmedium aus der neunten Zone 13 wird der Spritzvorrichtung der dritten Zone 7 zugeführt. Behandlungsmedium aus der zehnten Zone 14 wird der Spritzvorrichtung der zweiten Zone 6 zugeführt. Behandlungsmedium aus der elften Zone 15 wird der Spritzvorrichtung der ersten Zone 6 zugeführt. Behandlungsmedium aus der zwölften Zone 16 wird der Spritzvorrichtung der zwölften Zone 16 zugeführt.

In den drei Pasteurisierungszonen 22-24 wird das Behandlungsmedium aus einer der Zonen der jeweiligen Zone wieder zugeführt. Es findet kein Austausch von Behandlungsmedium zwischen diesen Zonen 22-24 statt.

Eine Rekuperation von Wärme des Behandlungsmediums kann dadurch stattfinden, dass das Behandlungsmedium aus der ersten Zone 5 der Spritzvorrichtung der elften Zone 15 zugeführt wird, dass das Behandlungsmedium aus der zweiten Zone 6 der Spritzvorrichtung der zehnten Zone 14 zugeführt wird, dass das Behandlungsmedium aus der dritten Zone 7 der Spritzvorrichtung der neunten Zone 13 zugeführt wird und dass das Behandlungsmedium aus der vierten Zone 8 der Spritzvorrichtung der achten Zone 12 zugeführt wird.

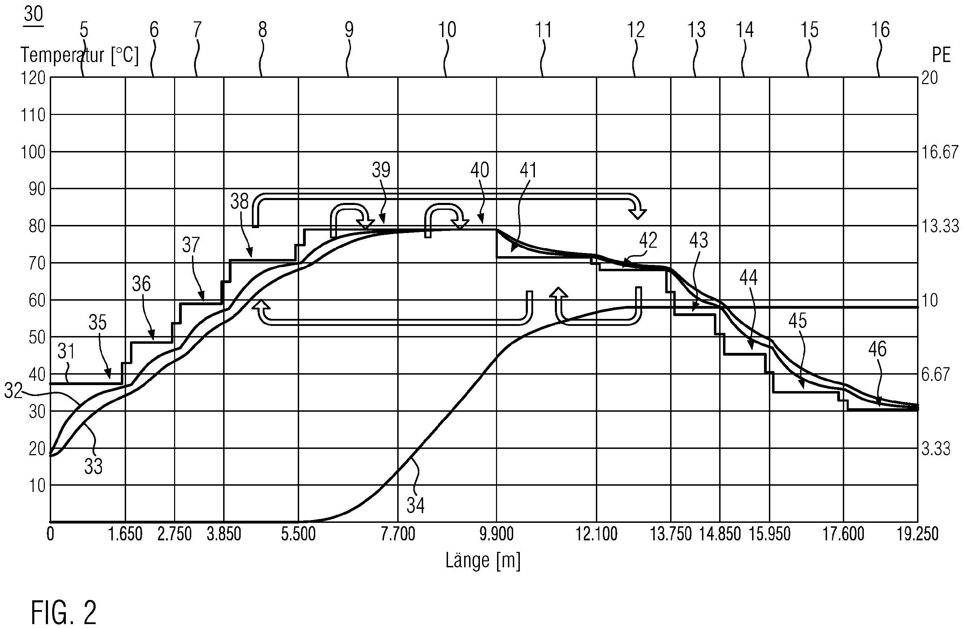

Die Figur 2 zeigt ein Diagramm 30 mit Temperaturkurven 31, 32, 33 einer Pasteurisation und einer Kurve 34 der aufgenommenen Pasteurisierungseinheiten PE bei 80% Transportgeschwindigkeit. 80% Transportgeschwindigkeit kann hierbei eine Transportgeschwindigkeit bedeuten, die 80% der Nenntransportgeschwindigkeit entsprechen kann. 80% Transportgeschwindigkeit kann während eines Betriebs des Pasteurs erforderlich sein, wenn Störungen auftreten. Störungen können auftreten, wenn eine dem Pasteur vorgeschaltete Maschine zu wenige Behälter (im Vergleich zu einer Anzahl von Behältern bei Normalbetrieb) an den Pasteur übergeben kann und/oder wenn eine dem Pasteur nachgeschaltete Maschine zu wenige Behälter (im Vergleich zu einer Anzahl von Behältern bei Normalbetrieb) abtransportieren kann umfassen.

Die Regelung der Transportgeschwindigkeit in dem Pasteur, kann bei einem Reduzieren der Transportgeschwindigkeit von 100% auf 80% durch ein Reduzieren der Anzahl der Pasteurisierungszonen des Pasteurs, die bei 100% Transportgeschwindigkeit vorgesehen sind, um eine Zone und ein Erhöhen der Anzahl der Kühlzonen des Pasteurs um entsprechend eine Zone erfolgen. Dies kann durch Umschaltung von einem oder mehreren Ventilen, beispielsweise Klappenventilen, erfolgen, die in Rohrleitungen zwischen den Zonen und den Spritzvorrichtungen angeordnet sein können. Bei 80% Transportgeschwindigkeit dienen die ersten vier Zonen 5-7, 8 wie bei 100% Transportgeschwindigkeit als Anwärmzonen 35, 36, 37, 38, die fünfte und sechste Zone 9, 10 dienen jeweils als Pasteurisierungszone 39, 40 und die siebte bis zwölfte Zone 12-16 dienen jeweils als Kühlzone 41, 42, 43, 44, 45, 46.

Die dritte Pasteurisierungszone, die in der Figur 1 beschrieben wurde, wurde somit in die erste Kühlzone umgewandelt, sodass nun sechs Kühlzonen 41-46 vorhanden sind, dafür aber nur noch zwei Pasteurisierungszonen 39, 40. Die Reduktion der Anzahl der Pasteurisierungszonen und die entsprechende Erhöhung der Anzahl der Kühlzonen kann durch Einbindung der dritten Pasteurisierungszone der Figur 1 in eine Spritzkaskade durch Öffnen bzw. Schließen entsprechender Ventile erfolgen. Diese Ventile können in der Druckverrohrung der entsprechenden Spritzvorrichtung vorgesehen sein, um einen ausreichenden Druck des Behandlungsmediums und damit ein einheitliches Spritzbild der Spritzvorrichtungen zu gewährleisten.

Zunächst werden wie bei 100% Transportgeschwindigkeit die vier Anwärmzonen 35-38 von Behältern nacheinander passiert. Die Anzahl der Anwärmzonen 35-38 wird bei der Reduzierung der Transportgeschwindigkeit nicht verändert. Die Behandlungstemperatur des Behandlungsmediums in den einzelnen Anwärmzonen für die Spritzvorrichtungen verwendet wird, ist bei 80% Transportgeschwindigkeit jedoch verschieden zu den Temperaturwerten bei 100% Transportgeschwindigkeit. In der ersten Anwärmzone 35 kann eine Temperatur des Behandlungsmediums, das auf die Behälter aufgespritzt werden kann, bei 37,5 °C liegen, in der zweiten Anwärmzone 36 bei 48,5 °C, in der dritten Anwärmzone 37 bei 59,0 °C und in der vierten Anwärmzone 38 bei 70,8 °C.

Auf die vier Anwärmzonen 35-38 folgen die zwei Pasteurisierungszonen 39, 40, in denen die Behandlungstemperatur des Behandlungsmediums, das für die Spritzvorrichtung verwendet wird, in den beiden Pasteurisierungszonen 39, 40 einer Pasteurisierungstemperatur entspricht, die beispielsweise 79,0 °C betragen kann. An der Kurve 34 der aufgenommenen Pasteurisierungseinheiten PE ist die Zunahme der aufgenommenen Pasteurisierungseinheiten PE zu erkennen, die in der ersten Pasteurisierungszone 39 beginnt und sich in der zweiten Pasteurisierungszone 40 kontinuierlich fortsetzt. Die aufgenommenen Pasteurisierungseinheiten sind dabei in der Einheit PE über der im Pasteur zurückgelegten Länge in Meter angegeben.

Auf die zwei Pasteurisierungszonen 39, 40 folgen die sechs Kühlzonen 41-46, wobei die Behandlungstemperatur des Behandlungsmediums, das für die Spritzvorrichtung verwendet wird, in den aufeinanderfolgenden Kühlzonen 41-46 stufenförmig abnimmt. In der ersten Kühlzone 41 kann eine Temperatur des Behandlungsmediums, das auf die Behälter aufgespritzt werden kann, bei 71,5 °C liegen, in der zweiten Kühlzone 42 bei 68,1 °C, in der dritten Kühlzone 43 bei 56,1 °C, in der vierten Kühlzone 44 bei 45,5 °C, in der fünften Kühlzone 45 bei 35,2 °C und in der sechsten Kühlzone 46 bei 30,7 °C. In der ersten Kühlzone 41 und der zweiten Kühlzone 42 können durch die im Behälter vorherrschenden Temperaturen weiter Pasteurisierungseinheiten PE aufgenommen werden, erst gegen Ende der zweiten Kühlzone 42 bleibt dann die Anzahl der aufgenommenen Pasteurisierungseinheiten konstant. Die nach der Pasteurisierung eines Behälters erreichten Pasteurisierungseinheiten PE können einen Wert von 9,7 haben.

In dem Diagramm ist der Kurvenverlauf der Behandlungstemperatur für die Spritzvorrichtungen mit dem Bezugszeichen 31 gekennzeichnet. Die Behandlungstemperatur ist dabei in Grad Celsius über der im Pasteur zurückgelegten Länge in Meter angegeben.

Zudem ist der Temperaturverlauf des Produkts im Innern der Behälter, an einem Punkt in einem Drittel der Höhe der Behälter von Boden aus gemessen, in der Kurve mit dem Bezugszeichen 33 angegeben. Die Produkttemperatur ist dabei in Grad Celsius über der im Pasteur zurückgelegten Länge in Meter angegeben.

Weiter ist mit dem Bezugszeichen 32 der Kurvenverlauf der mittleren Temperatur dargestellt. Die mittlere Temperatur ist dabei in Grad Celsius über der im Pasteur zurückgelegten Länge in Meter angegeben.

Für die Verteilung des Behandlungsmediums zu den Spritzvorrichtungen der jeweiligen Zonen des Pasteurs ist vorgesehen, dass Behandlungsmedium aus der achten Zone 12 der Spritzvorrichtung der siebten Zone 11 zugeführt wird, dass Behandlungsmedium aus der siebten Zone 11 der Spritzvorrichtung der vierten Zone 8 zugeführt wird, dass Behandlungsmedium aus der neunten Zone 13 der Spritzvorrichtung der dritten Zone 7 zugeführt wird, dass Behandlungsmedium aus der zehnten Zone 14 der Spritzvorrichtung der zweiten Zone 6 zugeführt wird, dass Behandlungsmedium aus der elften Zone 15 der Spritzvorrichtung der ersten Zone 5 zugeführt wird und dass das Behandlungsmedium aus der zwölften Zone 16 der Spritzvorrichtung der zwölften Zone 16 zugeführt wird.

In den zwei Pasteurisierungszonen 39, 40 wird das Behandlungsmedium aus einer der Zonen der jeweiligen Zone zugeführt. Es findet kein Austausch von Behandlungsmedium zwischen diesen Zonen statt.

Eine Rekuperation von Wärme des Behandlungsmediums kann dadurch stattfinden, dass das Behandlungsmedium aus der ersten Zone 5 der Spritzvorrichtung der elften Zone 15 zugeführt wird, dass das Behandlungsmedium aus der zweiten Zone 6 der Spritzvorrichtung der zehnten Zone 14 zugeführt wird, dass das Behandlungsmedium aus der dritten Zone 7 der Spritzvorrichtung der neunten Zone 13 zugeführt wird und dass das Behandlungsmedium aus der vierten Zone 8 der Spritzvorrichtung der achten Zone 12 zugeführt wird.

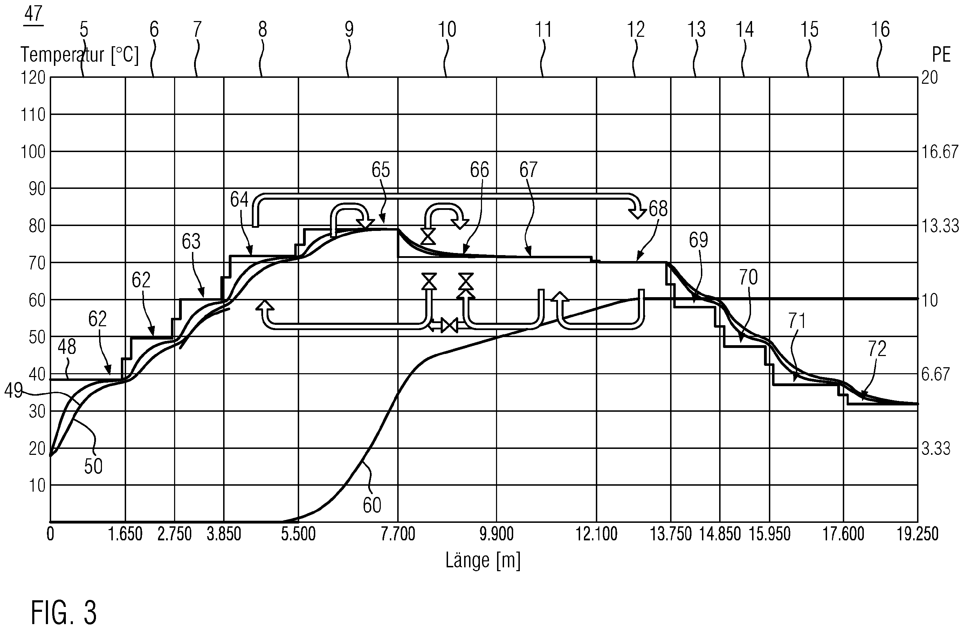

Die Figur 3 zeigt ein Diagramm 47 mit Temperaturkurven 48, 49, 50 einer Pasteurisation und einer Kurve 34 der aufgenommenen Pasteurisierungseinheiten PE bei 50% Transportgeschwindigkeit. 50% Transportgeschwindigkeit kann hierbei eine Transportgeschwindigkeit bedeuten, die 50% der Nenntransportgeschwindigkeit entsprechen kann. 50% Transportgeschwindigkeit kann während eines Betriebs des Pasteurs erforderlich sein, wenn Störungen, wie hinsichtlich der Figur 2 beschrieben, beispielsweise in verstärktem Maße auftreten.

Die Regelung der Transportgeschwindigkeit in dem Pasteur, kann bei einem Reduzieren der Transportgeschwindigkeit von 80% auf 50% durch ein weiteres Reduzieren der Anzahl der Pasteurisierungszonen des Pasteurs, die bei 80% Transportgeschwindigkeit vorgesehen sind, um eine Zone und ein Erhöhen der Anzahl der Kühlzonen des Pasteurs um entsprechend eine Zone erfolgen.

Die zweite Pasteurisierungszone, die in der Figur 2 beschrieben wurde, wurde somit in die erste Kühlzone umgewandelt, sodass nun sieben Kühlzonen 66, 67, 68, 69, 70, 71, 72 vorhanden sind, dafür aber nur noch eine Pasteurisierungszone 65. Die Reduktion der Anzahl der Pasteurisierungszonen und die entsprechend Erhöhung der Anzahl der Kühlzonen kann durch Einbindung der zweiten Pasteurisierungszone der Figur 2 in eine Spritzkaskade durch Öffnen bzw. Schließen entsprechender Ventile erfolgen. Diese Ventile können in der Druckverrohrung der entsprechenden Spritzvorrichtung vorgesehen sein, um einen ausreichenden Druck des Behandlungsmediums und damit ein einheitliches Spritzbild der Spritzvorrichtungen zu gewährleisten.

Zunächst werden wie bei 100% Transportgeschwindigkeit die vier Anwärmzonen 61, 62, 63, 64 nacheinander von den Behältern passiert. Die Anzahl der Anwärmzonen wird bei der Reduzierung der Transportgeschwindigkeit nicht verändert. Die Temperaturwerte der Behandlungstemperaturen des Behandlungsmediums in den einzelnen Zonen ist bei 50% Transportgeschwindigkeit jedoch verschieden zu den Temperaturwerten bei 80% Transportgeschwindigkeit. In der ersten Anwärmzone 61 kann eine Temperatur des Behandlungsmediums, das auf die Behälter aufgespritzt werden kann, bei 38,6 °C liegen, in der zweiten Anwärmzone 62 bei 49,4 °C, in der dritten Anwärmzone 63 bei 60,1 °C und in der vierten Anwärmzone 64 bei 71,5 °C.

Auf die vier Anwärmzonen 61-64 folgt eine Pasteurisierungszone 65, in der die Behandlungstemperatur des Behandlungsmediums einer Pasteurisierungstemperatur entspricht, die beispielsweise 78,8 °C betragen kann. An der Kurve 60 der aufgenommenen Pasteurisierungseinheiten PE ist die Zunahme der aufgenommenen Pasteurisierungseinheiten PE zu erkennen, die in der Pasteurisierungszone 65 beginnt. Die aufgenommenen Pasteurisierungseinheiten ist dabei in der Einheit PE über der im Pasteur zurückgelegten Länge in Meter angegeben

Auf die eine Pasteurisierungszone 65 folgen sieben Kühlzonen 66-72, wobei die Behandlungstemperaturen des Behandlungsmediums das für die Spritzvorrichtung verwendet wird, in den aufeinanderfolgenden Abkühlzonen 66-72 stufenförmig abnimmt. In der ersten Kühlzone 66 kann eine Temperatur des Behandlungsmediums, das auf die Behälter aufgespritzt werden kann, bei 71,3 °C liegen, in der zweiten Kühlzone 67 bei 70,8 °C, in der dritten Kühlzone 68 bei 70 °C, in der vierten Kühlzone 69 bei 58,2 °C, in der fünften Kühlzone 70 bei 47,5 °C, in der sechsten Kühlzone 71 bei 37,3 °C und in der siebten Kühlzone 46 bei 31,6 °C. In der ersten Kühlzone 66, der zweiten Kühlzone 67 und der dritten Kühlzone 68 können durch die im Behälter vorherrschenden Temperaturen weiter Pasteurisierungseinheiten PE aufgenommen werden, erst gegen Ende der dritten Kühlzone 68 bleibt dann die Anzahl der aufgenommenen Pasteurisierungseinheiten konstant. Die nach der Pasteurisierung eines Behälters erreichten Pasteurisierungseinheiten PE können einen Wert von 10,1 haben.

In dem Diagramm ist der Kurvenverlauf der Behandlungstemperatur für die Spritzvorrichtungen mit dem Bezugszeichen 48 gekennzeichnet. Die Behandlungstemperatur ist dabei in Grad Celsius über der im Pasteur zurückgelegten Länge in Meter angegeben.

Zudem ist der Temperaturverlauf des Produkts im Innern der Behälter, an einem Punkt in einem Drittel der Höhe der Behälter von Boden aus gemessen, in der Kurve mit dem Bezugszeichen 50 angegeben. Die Produkttemperatur ist dabei in Grad Celsius über der im Pasteur zurückgelegten Länge in Meter angegeben.

Weiter ist mit dem Bezugszeichen 49 der Kurvenverlauf der mittleren Temperatur dargestellt. Die mittlere Temperatur ist dabei in Grad Celsius über der im Pasteur zurückgelegten Länge in Meter angegeben.

Für die Verteilung des Behandlungsmediums zu den Spritzvorrichtungen der jeweiligen Zonen des Pasteurs ist vorgesehen, dass Behandlungsmedium aus der achten Zone 12 der Spritzvorrichtung der siebten Zone 11 zugeführt wird, dass Behandlungsmedium aus der siebten Zone 11 der Spritzvorrichtung der sechsten Zone 10 und/oder der vierten Zone 8 zugeführt wird, dass Behandlungsmedium aus der sechsten Zone 10 der Spritzvorrichtung der vierten Zone 8 zugeführt wird, dass Behandlungsmedium aus der neunten Zone 13 der Spritzvorrichtung der dritten Zone 7 zugeführt wird, dass Behandlungsmedium aus der zehnten Zone 14 der Spritzvorrichtung der zweiten Zone 6 zugeführt wird, dass Behandlungsmedium aus der elften Zone 15 der Spritzvorrichtung der ersten Zone 5 zugeführt wird und dass das Behandlungsmedium aus der zwölften Zone 16 der Spritzvorrichtung der zwölften Zone 16 zugeführt wird.

In der einen Pasteurisierungszone 65 wird das Behandlungsmedium aus der Pasteurisierungszone 65 der Spritzvorrichtung dieser Zone wieder zugeführt.

In der sechsten Zone 10 wird das Behandlungsmedium aus der sechsten Zone 10 der Spritzvorrichtung der sechsten Zone 10 zugeführt.

Eine Rekuperation von Wärme des Behandlungsmediums kann dadurch stattfinden, dass das Behandlungsmedium aus der ersten Zone 5 der Spritzvorrichtung der elften Zone 15 zugeführt wird, dass das Behandlungsmedium aus der zweiten Zone 6 der Spritzvorrichtung der zehnten Zone 14 zugeführt wird, dass das Behandlungsmedium aus der dritten Zone 7 der Spritzvorrichtung der neunten Zone 13 zugeführt wird und dass das Behandlungsmedium aus der vierten Zone 8 der Spritzvorrichtung der achten Zone 12 zugeführt wird.

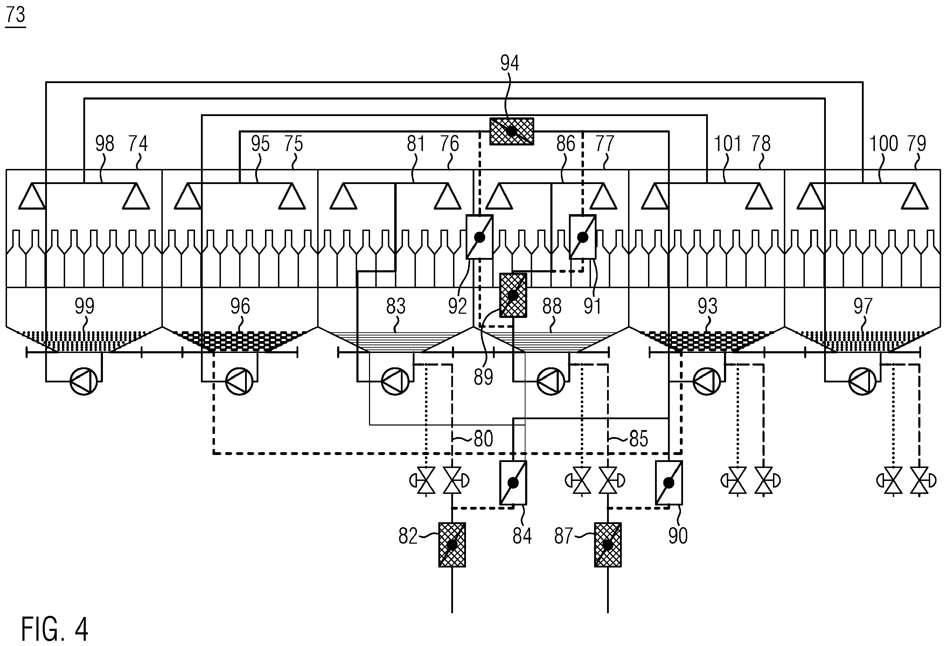

Die Figur 4 zeigt eine Schaltanordnung 73 einer Spritzkaskade bei 100% Transportgeschwindigkeit in einem Pasteur, der zwei Anwärmzonen 74, 75, zwei Pasteurisierungszonen 76, 77 und zwei Kühlzonen 78, 79 umfasst. Ein Betrieb mit 100% Transportgeschwindigkeit kann als Normalbetrieb angesehen werden.

Der Zuführleitung 80 der Spritzvorrichtung 81 der ersten Pasteurisierungszone 76 kann warmes Behandlungsmedium über ein geöffnetes erstes Ventil 82 zugeführt werden, das mit Behandlungsmedium 83 aus der ersten Pasteurisierungszone 76 gemischt werden und dann der Spritzvorrichtung 81 der ersten Pasteurisierungszone 76 zugeführt werden kann. Ein drittes Ventil 84 ist geschlossen. Das warme Behandlungsmedium kann durch einen Wärmetauscher bereitgestellt werden.

Der Zuführleitung 85 der Spritzvorrichtung 86 der zweiten Pasteurisierungszone 77 kann warmes Behandlungsmedium über ein geöffnetes zweites Ventil 87 zugeführt werden, das mit Behandlungsmedium 88 aus der zweiten Pasteurisierungszone 77 gemischt werden kann, und über ein geöffnetes fünftes Ventil 89 dann der Spritzvorrichtung 86 der zweiten Pasteurisierungszone 77 zugeführt werden kann. Ein viertes Ventil 90, ein sechstes Ventil 91 und ein siebtes Ventil 92 sind geschlossen. Das warme Behandlungsmedium kann durch den Wärmetauscher oder einen weiteren Wärmetauscher bereitgestellt werden.

Aus der ersten Kühlzone 78 wird Behandlungsmedium 93 über ein geöffnetes achtes Ventil 94 der Spritzvorrichtung 95 der zweiten Anwärmzone 75 zugeführt.

Aus der zweiten Anwärmzone 75 wird Behandlungsmedium 96 der Spritzvorrichtung 101 ersten Kühlzone 78 zugeführt.

Aus der zweiten Kühlzone 79 wird Behandlungsmedium 97 der Spritzvorrichtung 98 der ersten Anwärmzone 74 zugeführt.

Aus der ersten Anwärmzone 74 wird Behandlungsmedium 99 der Spritzvorrichtung 100 der zweiten Kühlzone 79 zugeführt.

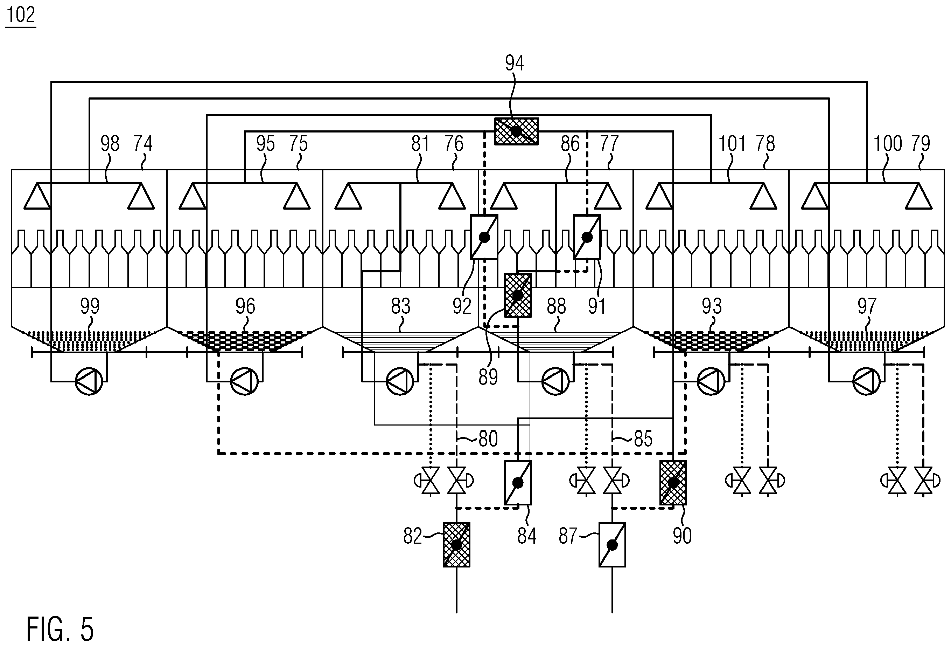

Die Figur 5 zeigt eine Schaltanordnung 102 der Spritzkaskade des in der Figur 4 beschriebenen Pasteurs, wobei die zweite Pasteurisierungszone 77 gekühlt wird.

Der Zuführleitung 80 der Spritzvorrichtung 81 der ersten Pasteurisierungszone 76 kann warmes Behandlungsmedium über das geöffnete erste Ventil 82 zugeführt werden, das mit Behandlungsmedium 83 aus der ersten Pasteurisierungszone 76 gemischt werden und dann der Spritzvorrichtung 81 der ersten Pasteurisierungszone 76 zugeführt werden kann. Das warme Behandlungsmedium kann durch den Wärmetauscher bereitgestellt werden.

Der Zuführleitung 103 der Spritzvorrichtung 95 der zweiten Anwärmzone 75 kann warmes Behandlungsmedium über das geöffnete vierte Ventil 90 zugeführt werden, das mit Behandlungsmedium 93 aus der ersten Kühlzone 78 gemischt werden kann, und über das geöffnete achte Ventil 94 dann der Spritzvorrichtung 95 der zweiten Anwärmzone 75 zugeführt werden kann. Das warme Behandlungsmedium kann durch den Wärmetauscher oder den weiteren Wärmetauscher bereitgestellt werden.

Aus der zweiten Anwärmzone 75 wird Behandlungsmedium 96 der ersten Kühlzone 78 zugeführt.

Behandlungsmedium 88 aus der zweiten Pasteurisierungszone 77 kann über das geöffnete fünfte Ventil 89 der Spritzvorrichtung 86 der zweiten Pasteurisierungszone 77 zugeführt werden.

Aus der zweiten Kühlzone 79 wird Behandlungsmedium 97 der Spritzvorrichtung 98 der ersten Anwärmzone 74 zugeführt.

Aus der ersten Anwärmzone 74 wird Behandlungsmedium 99 der Spritzvorrichtung 100 der zweiten Kühlzone 79 zugeführt.

Das zweite Ventil 87, dritte Ventil 84, sechste Ventil 91 und siebte Ventil 92 sind geschlossen.

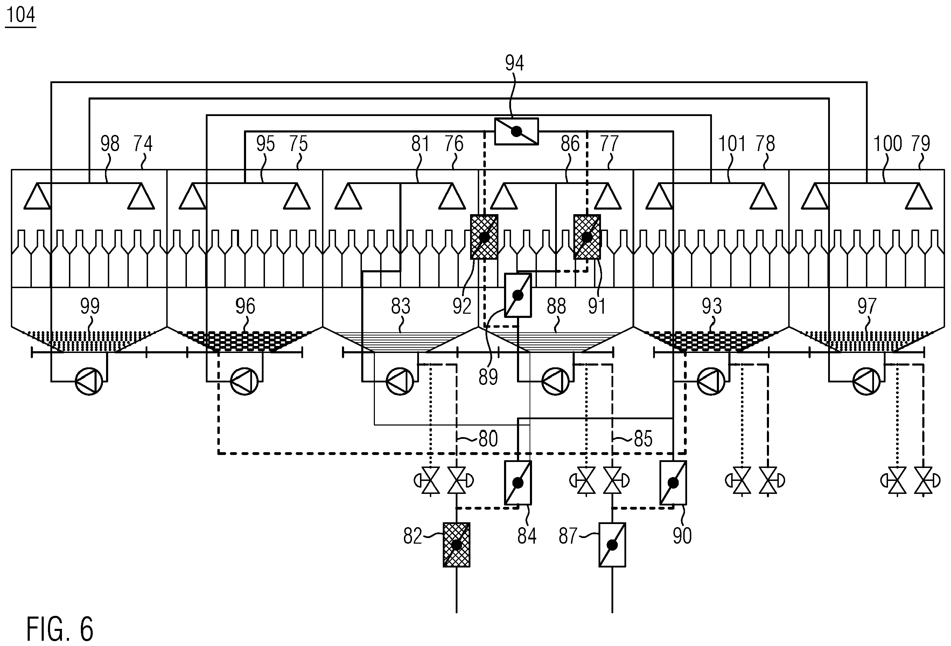

Die Figur 6 zeigt eine Schaltanordnung 104 des in der Figur 4 beschriebenen Pasteurs, wobei die zweite Pasteurisierungszone 77 mittels der ersten Kühlzone 78 gekühlt wird.

Der Zuführleitung 80 der Spritzvorrichtung 81 der ersten Pasteurisierungszone 76 kann warmes Behandlungsmedium über das geöffnete erste Ventil 82 zugeführt werden, das mit Behandlungsmedium 83 aus der ersten Pasteurisierungszone 76 gemischt werden und dann der Spritzvorrichtung 81 der ersten Pasteurisierungszone 76 zugeführt werden kann. Das warme Behandlungsmedium kann durch den Wärmetauscher bereitgestellt werden.

Behandlungsmedium 88 aus der zweiten Pasteurisierungszone 77 kann über das geöffnete siebte Ventil 92 der Spritzvorrichtung 95 der zweiten Anwärmzone 75 zugeführt werden.

Behandlungsmedium 96 aus der zweiten Anwärmzone 75 kann der Spritzvorrichtung 101 ersten Kühlzone 78 zugeführt werden.

Behandlungsmedium 93 aus der ersten Kühlzone 78 kann über das geöffnete sechste Ventil 91 der Spritzvorrichtung 86 der zweiten Pasteurisierungszone 77 zugeführt werden.

Aus der zweiten Kühlzone 79 wird Behandlungsmedium 97 der Spritzvorrichtung 98 der ersten Anwärmzone 74 zugeführt.

Aus der ersten Anwärmzone 74 wird Behandlungsmedium 99 der Spritzvorrichtung 100 der zweiten Kühlzone 79 zugeführt.

Das zweite Ventil 87, dritte Ventil 84, vierte Ventil 90 und achte Ventil 94 sind geschlossen.

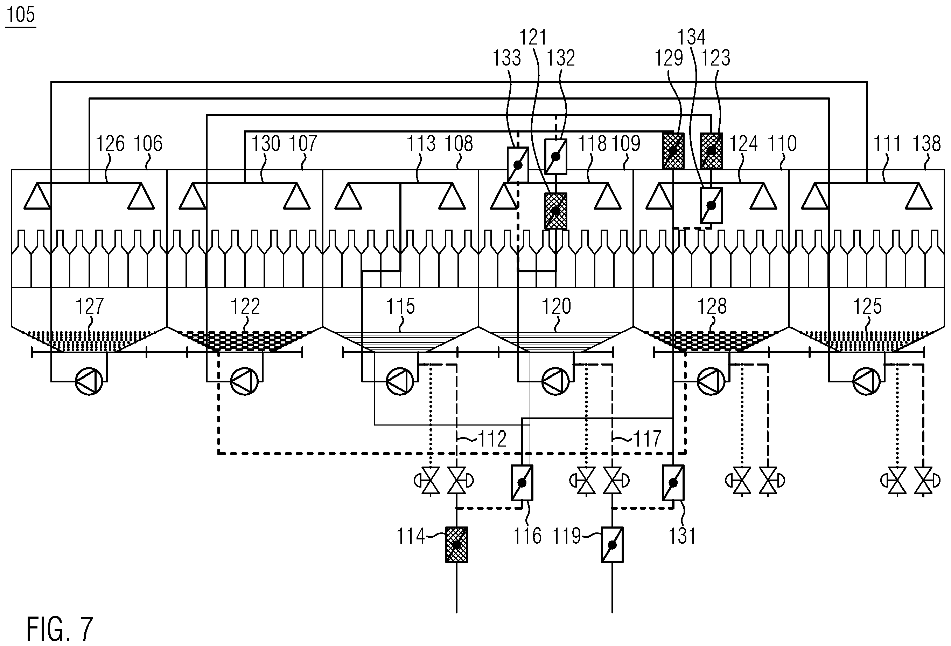

Die Figur 7 zeigt eine Schaltanordnung 105 für eine Verschiebung einer Rekuperationszone bei 100% Transportgeschwindigkeit in einem Pasteur, der zwei Anwärmzonen 106, 107, zwei Pasteurisierungszonen 108, 109 und zwei Kühlzonen 110, 111 umfasst. Ein Betrieb mit 100% Transportgeschwindigkeit kann als Normalbetrieb angesehen werden.

Der Zuführleitung 112 der Spritzvorrichtung 113 der ersten Pasteurisierungszone 108 kann warmes Behandlungsmedium über ein geöffnetes erstes Ventil 114 zugeführt werden, das mit Behandlungsmedium 115 aus der ersten Pasteurisierungszone 108 gemischt werden und dann der Spritzvorrichtung 113 der ersten Pasteurisierungszone 108 zugeführt werden kann. Ein drittes Ventil 116 ist geschlossen.

Der Zuführleitung 117 der Spritzvorrichtung 118 der zweiten Pasteurisierungszone 109 kann warmes Behandlungsmedium über ein geöffnetes zweites Ventil 119 zugeführt werden, das mit Behandlungsmedium 120 aus der zweiten Pasteurisierungszone 109 gemischt werden kann, und über ein geöffnetes fünftes Ventil 121 dann der Spritzvorrichtung 118 der zweiten Pasteurisierungszone 109 zugeführt werden kann.

Aus der ersten Kühlzone 110 wird Behandlungsmedium 128 über ein geöffnetes neuntes Ventil 129 der Spritzvorrichtung 130 der zweiten Anwärmzone 107 zugeführt.

Aus der zweiten Anwärmzone 107 wird Behandlungsmedium 122 über ein geöffnetes achtes Ventil 123 der Spritzvorrichtung 124 ersten Kühlzone 110 zugeführt.

Aus der zweiten Kühlzone 111 wird Behandlungsmedium 125 der Spritzvorrichtung 126 der ersten Anwärmzone 106 zugeführt.

Aus der ersten Anwärmzone 106 wird Behandlungsmedium 127 der Spritzvorrichtung 138 der zweiten Kühlzone 111 zugeführt.

Ein viertes Ventil 131, ein sechstes Ventil 132, ein siebtes Ventil 133 und ein zehntes Ventil 134 sind geschlossen.

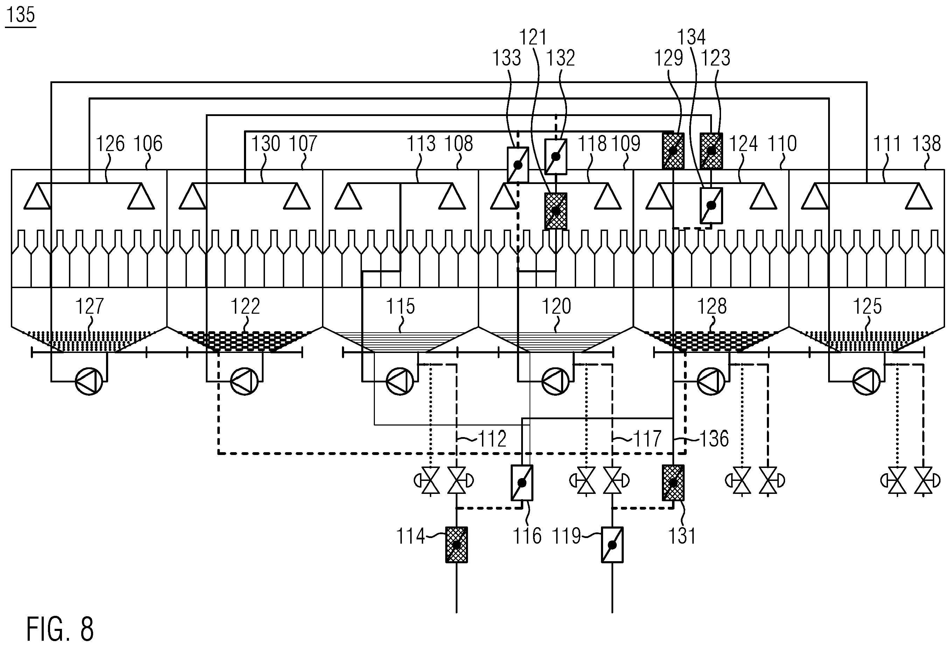

Die Figur 8 zeigt eine Schaltanordnung 135 für eine Verschiebung der Rekuperationszone bei 80% Transportgeschwindigkeit in dem Pasteur, der in der Figur 7 beschrieben wurde.

Der Zuführleitung 112 der Spritzvorrichtung 113 der ersten Pasteurisierungszone 108 kann warmes Behandlungsmedium über das geöffnete erste Ventil 114 zugeführt werden, das mit Behandlungsmedium 115 aus der ersten Pasteurisierungszone 108 gemischt werden und dann der Spritzvorrichtung 113 der ersten Pasteurisierungszone 108 zugeführt werden kann. Das drittes Ventil 116 ist geschlossen.

Aus der zweiten Pasteurisierungszone 109 wird Behandlungsmedium 120 über das geöffnete fünfte Ventil 121 der Spritzvorrichtung 118 der zweiten Pasteurisierungszone 109 zugeführt.

Einer Abführleitung 136 der ersten Kühlzone 110 kann warmes Behandlungsmedium über das geöffnete vierte Ventil 131 zugeführt werden, das mit Behandlungsmedium 128 aus der ersten Kühlzone 110 gemischt werden kann, und über das geöffnete neunte Ventil 129 dann der Spritzvorrichtung 130 der zweiten Anwärmzone 107 zugeführt werden kann.

Aus der zweiten Anwärmzone 107 wird Behandlungsmedium 122 über das geöffnete achte Ventil 123 der Spritzvorrichtung 124 ersten Kühlzone 110 zugeführt.

Aus der zweiten Kühlzone 111 wird Behandlungsmedium 125 der Spritzvorrichtung 126 der ersten Anwärmzone 106 zugeführt.

Aus der ersten Anwärmzone 106 wird Behandlungsmedium 127 der Spritzvorrichtung 138 der zweiten Kühlzone 111 zugeführt.

Das zweite Ventil 119, das sechste Ventil 132, das siebte Ventil 133 und das zehntes Ventil 134 sind geschlossen.

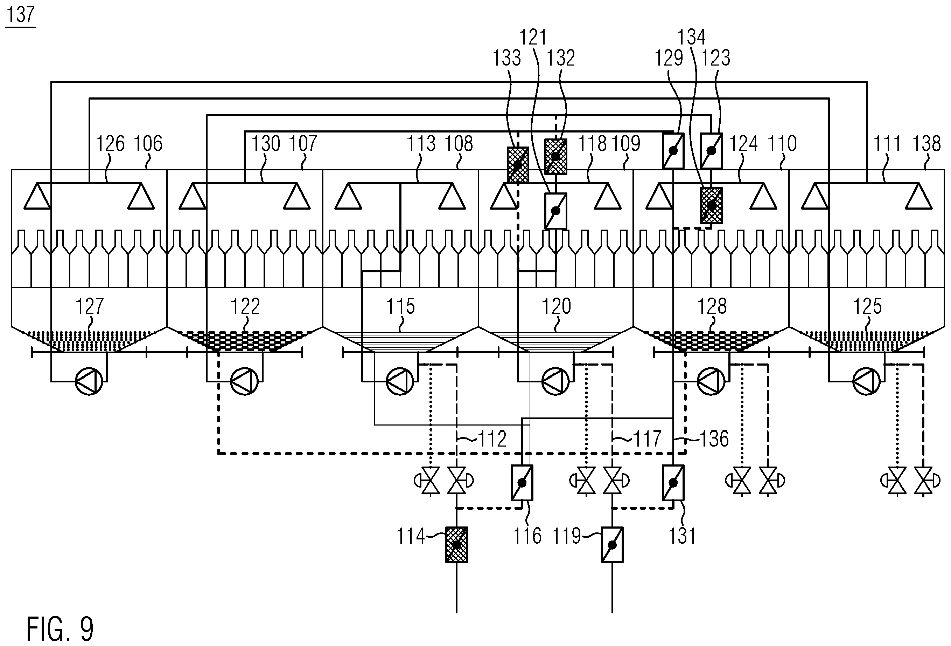

Die Figur 9 zeigt eine Schaltanordnung für eine Verschiebung der Rekuperationszone bei 50% Transportgeschwindigkeit in dem Pasteur, der in der Figur 7 beschrieben wurde.

Der Zuführleitung 112 der Spritzvorrichtung 113 der ersten Pasteurisierungszone 108 kann warmes Behandlungsmedium über das geöffnete erste Ventil 114 zugeführt werden, das mit Behandlungsmedium 115 aus der ersten Pasteurisierungszone 108 gemischt werden und dann der Spritzvorrichtung 113 der ersten Pasteurisierungszone 108 zugeführt werden kann. Das dritte Ventil 116 ist geschlossen.

Aus der zweiten Pasteurisierungszone 109 wird Behandlungsmedium 120 über das geöffnete siebte Ventil 133 der Spritzvorrichtung 130 der zweiten Anwärmzone 107 zugeführt.

Aus der zweiten Anwärmzone 107 wird Behandlungsmedium 122 über das geöffnete sechste Ventil 132 der Spritzvorrichtung 118 zweiten Pasteurisierungszone 109 zugeführt.

Aus der ersten Kühlzone 110 wird Behandlungsmedium 128 über das geöffnete zehnte Ventil 134 der Spritzvorrichtung 124 der ersten Kühlzone 110 zugeführt.

Aus der zweiten Kühlzone 111 wird Behandlungsmedium 125 der Spritzvorrichtung 126 der ersten Anwärmzone 106 zugeführt.

Aus der ersten Anwärmzone 106 wird Behandlungsmedium 127 der Spritzvorrichtung 138 der zweiten Kühlzone 111 zugeführt.

Das zweite Ventil 119, das achte Ventil 123 und das neunte Ventil 129 sind geschlossen.

## Patentansprüche

1. Verfahren zur Regelung einer Transportgeschwindigkeit in einem Pasteur, wobei das Verfahren umfasst:
- Regelung der Transportgeschwindigkeit durch ein Verändern, wie ein Reduzieren oder ein Erhöhen, der Transportgeschwindigkeit, entsprechendes erstes Verändern, wie Reduzieren oder Erhöhen, einer ersten Anzahl von Pasteurisierungszonen (22, 23, 24, 39, 40, 65, 76, 77, 108, 109) des Pasteurs um eine zweite Anzahl und entsprechendes zweites Verändern, wie Erhöhen oder Reduzieren, einer dritten Anzahl von Kühlzonen (25, 26, 27, 28, 29, 41, 42, 43, 44, 45, 46, 66, 67, 68, 69, 70, 71, 72, 78, 79, 110, 111) des Pasteurs um die zweite Anzahl.

2. Das Verfahren nach Anspruch 1, weiter umfassend:
- Beibehalten einer vierten Anzahl von Anwärmzonen (18, 19, 20, 21, 35, 36, 37, 38, 61, 62, 63, 64, 74, 75, 106, 107) des Pasteurs.

3. Das Verfahren nach Anspruch 1 oder 2, wobei das Verändern ein Reduzieren der Transportgeschwindigkeit, das entsprechende erste Verändern ein Reduzieren der ersten Anzahl von Pasteurisierungszonen (22, 23, 24, 39, 40, 65, 76, 77, 108, 109) des Pasteurs um die zweite Anzahl und das entsprechende zweite Verändern ein Erhöhen der dritten Anzahl von Kühlzonen (25, 26, 27, 28, 29, 41, 42, 43, 44, 45, 46, 66, 67, 68, 69, 70, 71, 72, 78, 79, 110, 111) des Pasteurs um die zweite Anzahl umfasst.

4. Das Verfahren nach Anspruch 1, wobei das Reduzieren der ersten Anzahl von Pasteurisierungszonen (22, 23, 24, 39, 40, 65, 76, 77, 108, 109) des Pasteurs um die zweite Anzahl und das Erhöhen der dritten Anzahl von Kühlzonen des Pasteurs um die zweite Anzahl umfasst:
- kontinuierliches Absenken einer ersten Temperatur in der letzten Pasteurisierungszone, wodurch die letzte Pasteurisierungszone zu einer ersten Kühlzone wird.

5. Das Verfahren nach Anspruch 4, wobei das kontinuierliche Absenken einer ersten Temperatur in der letzten Pasteurisierungszone umfasst:
- Umschaltung einer Versorgung einer Spritzungsvorrichtung in der letzten Pasteurisierungszone mit Behandlungsmedium aus der letzten Pasteurisierungszone in eine Versorgung der Spritzungsvorrichtung mit Behandlungsmedium aus der ersten Kühlzone und
- Umschaltung einer Versorgung einer Spritzungsvorrichtung in der letzten Aufwärmzone mit Behandlungsmedium aus der ersten Kühlzone in eine Versorgung der Spritzungsvorrichtung mit Behandlungsmedium aus der letzten Pasteurisierungszone.

6. Das Verfahren nach Anspruch 5, weiter umfassend:
- Unterbrechen einer separaten Zufuhr von Warmwasser in die letzte Pasteurisierungszone.

7. Das Verfahren nach einem der Ansprüche 3 bis 6, wobei das Reduzieren der ersten Anzahl von Pasteurisierungszonen (22, 23, 24, 39, 40, 65, 76, 77, 108, 109) des Pasteurs um die zweite Anzahl und das Erhöhen der dritten Anzahl von Kühlzonen des Pasteurs um die zweite Anzahl mittels Regeln von mehreren regelbaren Ventilen (89, 91, 92, 94, 121, 123, 129, 132, 133, 134) erfolgt, wobei beispielsweise die mehreren regelbaren Ventile (89, 91, 92, 94, 121, 123, 129, 132, 133, 134) in einer Druckverrohrung von Spritzvorrichtungen angeordnet sind, wobei beispielsweise die mehreren regelbaren Ventile (89, 91, 92, 94, 121, 123, 129, 132, 133, 134) Klappenventile umfassen.

8. Das Verfahren nach Anspruch 1 oder 2, wobei das Verändern ein Erhöhen der Transportgeschwindigkeit, das entsprechende erste Verändern ein Erhöhen der ersten Anzahl von Pasteurisierungszonen des Pasteurs um die zweite Anzahl und das entsprechende zweite Verändern ein Reduzieren der dritten Anzahl von Kühlzonen des Pasteurs um die zweite Anzahl umfasst.

9. Das Verfahren nach Anspruch 8, wobei das Erhöhen der ersten Anzahl von Pasteurisierungszonen des Pasteurs um die zweite Anzahl und das Erhöhen der dritten Anzahl von Kühlzonen des Pasteurs um die zweite Anzahl umfasst:
- kontinuierliches Erhöhen einer ersten Temperatur in der ersten Kühlzone, wodurch die erste Kühlzone zu einer letzten Pasteurisierungszone wird.

10. Das Verfahren nach einem der Ansprüche 1 bis 9, wobei das Verändern der Transportgeschwindigkeit kontinuierlich, beispielsweise stufenlos, erfolgt oder wobei das Verändern der Transportgeschwindigkeit in 0,5%-, 1%- oder 1,5%-Schritten erfolgt.

11. Vorrichtung zum Ausführen des Verfahrens nach einem der Ansprüche 1 bis 10, wobei die Vorrichtung umfasst:
- einen Pasteur mit einer ersten Anzahl von Pasteurisierungszonen (22, 23, 24, 39, 40, 65, 76, 77, 108, 109) und einer dritten Anzahl von Kühlzonen (25, 26, 27, 28, 29, 41, 42, 43, 44, 45, 46, 66, 67, 68, 69, 70, 71, 72, 78, 79, 110, 111),
- eine Transportvorrichtung, die ausgebildet ist, Behälter mit einer Transportgeschwindigkeit in eine Transportrichtung durch den Pasteur zu transportieren,
- eine Steuerungsvorrichtung eingerichtet zum Verändern, wie Reduzieren oder Erhöhen, der Transportgeschwindigkeit, entsprechendes ersten Verändern, wie Reduzieren oder Erhöhen, der ersten Anzahl von Pasteurisierungszonen (22, 23, 24, 39, 40, 65, 76, 77, 108, 109) des Pasteurs um eine zweite Anzahl und entsprechendes zweites Verändern, wie Erhöhen oder Reduzieren, der dritten Anzahl von Kühlzonen des Pasteurs um die zweite Anzahl.

12. Die Vorrichtung nach Anspruch 11, wobei der Pasteur eine vierte Anzahl von Anwärmzonen (18, 19, 20, 21, 35, 36, 37, 38, 61, 62, 63, 64, 74, 75, 106, 107) umfasst.

13. Die Vorrichtung nach Anspruch 11 oder 12, wobei die Vorrichtung mehrere regelbare Ventile (89, 91, 92, 94, 121, 123, 129, 132, 133, 134) umfasst.

14. Die Vorrichtung nach einem der Ansprüche 11 bis 13, wobei die mehreren regelbaren Ventile (89, 91, 92, 94, 121, 123, 129, 132, 133, 134) in einer Druckverrohrung von Spritzvorrichtungen angeordnet sind, wobei beispielsweise die mehreren regelbaren Ventile (89, 91, 92, 94, 121, 123, 129, 132, 133, 134) Klappenventile umfassen.

15. Die Vorrichtung nach einem der Ansprüche 11 bis 14, weiter umfassend eine Umschaltvorrichtung zur Umschaltung einer Versorgung einer Spritzungsvorrichtung in der letzten Pasteurisierungszone mit Behandlungsmedium aus der letzten Pasteurisierungszone in eine Versorgung der Spritzungsvorrichtung mit Behandlungsmedium aus der ersten Kühlzone und weiter umfassend eine Umschaltvorrichtung zur Umschaltung einer Versorgung einer Spritzungsvorrichtung in der letzten Aufwärmzone mit Behandlungsmedium aus der ersten Kühlzone in eine Versorgung der Spritzungsvorrichtung mit Behandlungsmedium aus der letzten Pasteurisierungszone.

16. Die Vorrichtung nach einem der Ansprüche 11 bis 15, wobei die Vorrichtung weiter einen Wärmetauscher zum Wärmen von Behandlungsmedium umfasst.

17. Die Vorrichtung nach einem der Ansprüche 11 bis 16, wobei die Vorrichtung weiter einen weiteren Wärmetauscher zum Kühlen von Behandlungsmedium umfasst.
